# EUROPEAN PATENT APPLICATION

(11) **EP 4 154 878 A1**
(43) Date of publication of application: **29.03.2023**
(21) Application number: 21808105.7
(22) Date of filing: 17.05.2021
(51) Int. Cl.: A61K 31/343, A61K 31/192, A61P 31/18, A61P 31/14, A61P 31/12

(54) **APPLICATION OF ACTIVE INGREDIENT OF ROOT OF LIGULILOBE SAGE OR PHARMACEUTICALLY ACCEPTABLE SALT THEREOF IN PREPARING ANTIVIRAL DRUG**

(30) Priority: 18.05.2020 CN 202010420541; 18.05.2020 CN 202010419480; 18.05.2020 CN 202010420247; 13.04.2021 CN 202110393153
(71) Applicant: Southern Medical University, Guangzhou, Guangdong 510515 (CN)
(72) Inventor: LIU, Shuwen, Guangzhou, Guangdong 510515 (CN); YANG, Chan, Guangzhou, Guangdong 510515 (CN); CHENG, Chen, Guangzhou, Guangdong 510515 (CN); XU, Wei, Guangzhou, Guangdong 510515 (CN); PAN, Xiaoyan, Guangzhou, Guangdong 510515 (CN); XU, Xinfeng, Guangzhou, Guangdong 510515 (CN); WANG, Jinshen, Guangzhou, Guangdong 510515 (CN)
(74) Representative: Canzler & Bergmeier Patentanwälte Partnerschaft mbB
(86) International application number: PCT/CN2021/094045
(87) International publication number: WO 2021/233239

(57) **Abstract**

Disclosed is an application of an active ingredient of Root of Ligulilobe Sage or a pharmaceutically acceptable salt thereof in preparing an antiviral drug. The application of the active ingredient of Root of Ligulilobe Sage or the pharmaceutically acceptable salt thereof in preparing an anti-SARS-Cov-2 drug is proposed for the first time, and a novel treatment means for a novel coronavirus infected person having a chronic underlying disease is provided; the application expands the use range of the active ingredient of Root of Ligulilobe Sage, and especially provides a novel drug for treating pneumonia (COVID-19) caused by novel coronavirus infection in the case of the global SARS-CoV-2 epidemic.

## Description

### TECHNICAL FIELD

The present invention relates to the technical field of medicine, in particular to a use of an active ingredient of *Salvia miltiorrhiza* Bunge or a pharmaceutically acceptable salt thereof in preparation of an antiviral drug.

### BACKGROUND

The coronavirus disease 2019 (COVID-19), caused by severe acute respiratory syndrome coronavirus 2 (SARS-CoV-2), has broken out worldwide. Because of its extremely high rate of transmission and long incubation period, it has become the primary public health threat of the world. SARS-CoV-2 belongs to coronavirus β genus and is a linear single-stranded RNA-enveloped virus. The length of the genome of SARS-CoV-2 is about 30 kb, including 10 genes in total, which are ORF1ab, S, ORF3a, E, M, ORF6, ORF7a, ORF8, N and ORF10 respectively. The entering of SARS-CoV-2 is mediated by glycosylated Spike (S) protein, which plays the key role in viral infection. Therefore, the development of research on the structure of the S protein is the first step of drug development. The S protein is cleaved into S1 and S2 by the host cell protease, in which the entering of a virus is mediated by S1 in combination with receptor angiotensin converting enzyme 2 (ACE2) of the host cell, and the subunit S2 mediates viral cell membrane fusion. At present, it is considered that the protein S2 has at least three conformations: a natural conformation before fusion, an intermediate conformation before hairpin and a hairpin conformation after fusion. Through these different conformations, the fusion of the virus and the target cell is driven. Therefore, the S protein inhibitor can prevent the virus from entering the host cell, and the subunits S1 and S2 can be used as targets for antiviral drug screening. At present, there is no specific medicine for SARS-CoV-2, and there is no drug approved for marketing. No inhibitor against entering of SARS-CoV-2 of a targeting S protein from natural products has been reported.

*Salvia miltiorrhiza* Bunge is a perennial erect herb of Genus Salvia in Labiatae. Its root and rhizome can be used as medicine. According to the characteristics and physical and chemical properties of separated compounds, them are divided into two categories: lipophilic tanshinones and the hydrophilic salvianolic acid derivatives compounds.

The hydrophilic acid ingredient of *Salvia miltiorrhiza* Bunge mainly shows an antioxidant effect. Salvianolic acid B is one of the natural products with the strongest antioxidant activity, it can scavenge oxygen free radicals and inhibit lipid peroxidation, and at the same time, and it can also inhibit platelet aggregation and thrombosis, which has important pharmacological effects on heart, brain, liver, kidney and other organs. In 2013, Rao Zihe et al., disclosed application of salvianolic acid B in preparing Influenza Virus H5N1 resisting drugs in the Chinese patent CN103127050A, but the mechanism lies in that salvianolic acid B can effectively restrain activity of RNA polymerase subunit PAN protein of HSN1, thereby inhibiting the replication of HSN1, and a PAN protein expressed by the N-terminal nucleotide sequence of the RNA polymerase subtype PA subunit of H5N1 influenza virus is selected as a target protein.

Salvianic acid A is one of the main effective ingredients in hydrophilic ingredients of *Salvia miltiorrhiza* Bunge. It is a phenolic aromatic acid compound separated from *Salvia miltiorrhiza* Bunge in 1980, also known as salvianolic acid. The research on the pharmacological effects of the salvianic acid A mainly focuses on the exploration on the characteristics of its cardiovascular activity and the mechanism of action. It has the functions to dilate coronary artery, resist platelet aggregation, scavenge oxygen free radicals, and improve impaired memory.

The content of salvianolic acid C in *Salvia miltiorrhiza* Bunge is relatively low, and its pharmacological effects are rarely studied. Salvianolic acid C has relatively strong pharmacological effects in antioxidation, anti-liver injury, anti-tumor, anti-thrombosis, etc. In 2016, Wang Yuefei et al., disclosed the free radical scavenging activity of salvianolic acid C in patent CN106596432A. In 2017, Du Guanhua et al., disclosed Application of Salvianolic Acid C in Preparing Anti-stroke Drug in CN109985033A. In 2019, Fan Xiaohui et al., disclosed Application of Salvianolic Acid C in Preparing Drug for Protecting Ischemic Brain Tissue Damage in patent CN110101693A. In 2019, Tang Hongjin et al., disclosed Application of Salvianolic Acid C in Preparing Protein Tyrosine Phosphatase 1B Inhibitor and Drug for Preventing and/or Treating Type 2 Diabetes in patent CN109820889A.

The lipophilic tanshinones are a kind of lipophilic diterpene quinone compounds. At present, separated tanshinone compounds mainly include more than 10 tanshinone monomers including dihydrotanshinone I, cryptotanshinone, isocryptotanshinone, tanshinone I, tanshinone IIA, and tanshinone IIB.

The above several active ingredients of *Salvia miltiorrhiza* Bunge include hydrophilic phenolic acid compounds or lipophilic tanshinone compounds, and which have not been reported for the preparation of drugs for treatment of COVID-19.

### SUMMARY

The present invention aims to provide a new use of active ingredients of *Salvia miltiorrhiza* Bunge.

Another objective of the present invention is to provide a novel antiviral drug.

In order to achieve the above objectives, the present invention adopts the following technical solutions.

Provided is a use of an active ingredient of *Salvia miltiorrhiza* Bunge or a pharmaceutically acceptable salt thereof in preparation of an antiviral drug. The active ingredients of *Salvia miltiorrhiza* Bunge are salvianic acid A, salvianolic acid B, salvianolic acid C, dihydrotanshinone I (DHT) or a DHT analogue.

A molecular formula of the salvianic acid A is C₉H₁₀O₅, a molecular weight thereof is 198.17, and a structural formula thereof is

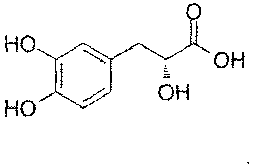

A molecular formula of the salvianolic acid B is C₃₆H₃₀O₁₆, a molecular weight thereof is 718.59, and a structural formula thereof is

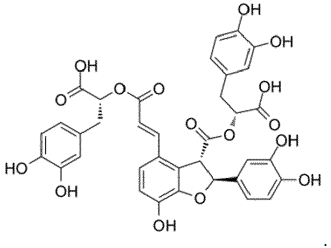

A molecular formula of the salvianolic acid C is C₂₆H₂₀O₁₀, a molecular weight thereof is 492.43, and a structural formula thereof is

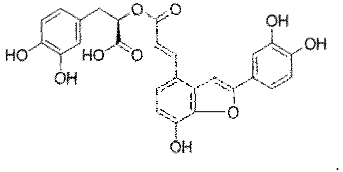

A molecular formula of the DHT is C₁₈H₁₄O₃, a molecular weight thereof is 278.31, and a structural formula thereof is

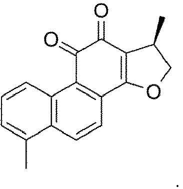

Provided is a use of an active ingredient of *Salvia miltiorrhiza* Bunge or a pharmaceutically acceptable salt thereof in preparation of a drug that inhibits a virus from entering a target cell. The active ingredients of *Salvia miltiorrhiza* Bunge are salvianic acid A, salvianolic acid B, salvianolic acid C, DHT or a DHT analogue.

Preferably, in the present invention, the DHT analogue is preferably Tanshinone I (T-A) or Cryptotanshinone (Cry-T).

We find that the salvianolic acid B has a certain inhibitory effect on a non-coronavirus or a coronavirus. Preferably, when the active ingredient of *Salvia miltiorrhiza* Bunge is the salvianolic acid B, the virus is preferably HIV-1, SARS-CoV, SARS-CoV-2 and MERS-CoV.

We find that the salvianic acid A, the salvianolic acid C, the DHT or the DHT analogue have obvious inhibitory effects on a coronavirus. Preferably, when the active ingredient of *Salvia miltiorrhiza* Bunge is the salvianic acid A, the salvianolic acid C, the DHT or the DHT analogue, the virus is a coronavirus.

In particular, we find that the DHT or the DHT analogue have obvious inhibitory effects on a variety of coronaviruses. Therefore, preferably, when the active ingredient of *Salvia miltiorrhiza* Bunge is the DHT or the DHT analogue, the virus is preferably HCoV-229E, HCoV-OC43, HCoV-NL63, HCoV-HKU1, Severe Acute Respiratory Syndrome Coronavirus SARS-CoV, Severe Acute Respiratory Syndrome Coronavirus 2 SARS-CoV-2 or Middle East Respiratory Syndrome Coronavirus MERS-CoV.

Experiments show that the active ingredient of *Salvia miltiorrhiza* Bunge in the present invention has a significant inhibitory effect on a virus that causes COVID-19. Therefore, most preferably, in the present invention, the virus is most preferably SARS-CoV-2.

Provided is a use of an active ingredient of *Salvia miltiorrhiza* Bunge or a pharmaceutically acceptable salt thereof in preparation of a drug that inhibits virus-cell fusion mediated by a viral S protein. The active ingredient of *Salvia miltiorrhiza* Bunge is salvianolic acid B.

Provided is a use of an active ingredient of *Salvia miltiorrhiza* Bunge or a pharmaceutically acceptable salt thereof in preparation of a drug that inhibits formation of a six-helix structure by fusion regions HR1 and HR2 of a subunit S2 of a viral S protein. The active ingredient of *Salvia miltiorrhiza* Bunge is salvianolic acid B.

Based on the above uses, we can combine the active ingredients of *Salvia miltiorrhiza* Bunge with pharmaceutically acceptable excipients to make an antiviral drug composition.

An antiviral pharmaceutical composition contains an active ingredient of *Salvia miltiorrhiza* Bunge or a pharmaceutically acceptable salt thereof as an active substance, and the active ingredient of *Salvia miltiorrhiza* Bunge is salvianic acid A, salvianolic acid B, salvianolic acid C, DHT or a DHT analogue.

A pharmaceutical composition for inhibiting a virus from entering a target cell contains an active ingredient of *Salvia miltiorrhiza* Bunge as an active substance or a pharmaceutically acceptable salt thereof, and the active ingredient of *Salvia miltiorrhiza* Bunge is salvianic acid A, salvianolic acid B, salvianolic acid C, DHT or a DHT analogue.

Preferably, when the active ingredient of *Salvia miltiorrhiza* Bunge is the salvianolic acid B, the virus is preferably HIV-1, SARS-CoV, SARS-CoV-2 and MERS-CoV.

Preferably, when the active ingredient of *Salvia miltiorrhiza* Bunge is the salvianic acid A, the salvianolic acid C, the DHT or the DHT analogue, the virus is a coronavirus.

Preferably, when the active ingredient of *Salvia miltiorrhiza* Bunge is the DHT or the DHT analogue, the virus is HCoV-229E, HCoV-OC43, HCoV-NL63, HCoV-HKU1, SARS-CoV, SARS-CoV-2 or MERS-CoV.

Most preferably, the virus is SARS-CoV-2.

A dosage form of the pharmaceutical composition of the present invention can be a common dosage form. Preferably, the pharmaceutical composition is an injection preparation or an oral preparation.

More preferably, the pharmaceutical composition is a set prescription preparation injection, a set prescription preparation tablet, a set prescription preparation capsule, a set prescription preparation pill or a set prescription preparation drop pill.

The oral preparation can be an oral capsule, a tablet or an oral liquid.

A pharmaceutical composition for inhibiting virus-cell fusion mediated by a viral S protein contains salvianolic acid B or a pharmaceutically acceptable salt thereof as an active substance.

A pharmaceutical composition for inhibiting formation of a six-helix structure by fusion regions HR1 and HR2 of a subunit S2 of a viral S protein contains salvianolic acid B as an active substance or a pharmaceutically acceptable salt thereof.

Preferably, the virus is HIV-1, SARS-CoV, SARS-CoV-2 and MERS-CoV.

Preferably, the pharmaceutical composition is an injection preparation or an oral preparation.

Preferably, the pharmaceutical composition is a set prescription preparation injection, a set prescription preparation tablet, a set prescription preparation capsule, a set prescription preparation pill or a set prescription preparation drop pill.

The present invention has the following beneficial effects.

The present invention is the first time to find a use of an active ingredient of *Salvia miltiorrhiza* Bunge such as salvianic acid A, salvianolic acid B, salvianolic acid C, DHT or a DHT analogue, or a pharmaceutically acceptable salt thereof in preparation of an antiviral drug. The use expands the usage range of the active ingredient of *Salvia miltiorrhiza* Bunge, especially for the preparation of anti-coronavirus drugs. In the context of COVID-19 pandemic that rages across the world, the present invention provides a certain feasibility basis for clinical Chinese medicine treatment of COVID-19 and provides a new drug to inhibit SARS-CoV-2.

Salvianic acid A and salvianolic acid B can also be used as therapeutic drugs for some chronic underlying diseases, for example, salvianolic acid B is suitable for patients with cardiovascular and cerebrovascular chronic underlying diseases such as atherosclerosis, etc. Therefore, the drug of the present invention can provide a safer treatment means for COVID-19 infected patients with chronic underlying diseases.

Experiments show that the salvianic acid A can effectively inhibit SARS-CoV-2 on an *in-vitro* culture cell Vero-E6, and a half effective concentration EC₅₀ thereof is 1.07 µM; the salvianic acid A is used for inhibiting the entering stage of SARS-CoV-2, and it can inhibit the infection of 293T/ACE2 cells by SARS-CoV-2 pseudovirus, and a half inhibitory concentration IC₅₀ thereof is 2.70 µM; and the salvianic acid A has no obvious cytotoxicity within the effective concentration range.

Experiments show that on an *in-vitro* culture cell Vero-E6 infection model, a half effective concentration EC₅₀ of the salvianolic acid B against SARS-CoV-2 activity is 55.47 µM; the salvianolic acid B can inhibit the entering stage of SARS-CoV-2, and it can inhibit the activity of the SARS-CoV-2 pseudovirus, and the IC₅₀ thereof is 0.416 µM. The salvianolic acid B has no obvious cytotoxicity within the effective concentration range.

Experiments show that the salvianolic acid C inhibits SARS-CoV-2 on an *in-vitro* culture cell Vero-E6, and a half effective concentration EC₅₀ thereof is 3.41 µM; the salvianolic acid C is used for inhibiting the entering stage of SARS-CoV-2, and it can inhibit the infection of 293T/ACE2 cells by SARS-CoV-2 pseudovirus, and a half effective inhibitory concentration IC₅₀ thereof is 5.90 µM; and the salvianolic acid C has no obvious cytotoxicity within the effective concentration range.

Experiments show that the DHT can effectively inhibit SARS-CoV-2 on an *in-vitro* culture cell Vero-E6, and a half effective concentration EC₅₀ thereof is 0.64 µM; the DHT is used for inhibiting the entering stage of SARS-CoV-2, and it can inhibit the infection of 293T/ACE2 cells by SARS-CoV-2 pseudovirus, and a half inhibitory concentration IC₅₀ thereof is 0.68 µM; in addition, the EC₅₀ of the T-A for inhibiting SARS-CoV-2 is 2.98 µM; and the EC₅₀ of the Cry-T for inhibiting SARS-CoV-2 is 1.46 µM. The DHT, the T-A and the Cry-T have no obvious cytotoxicity within the effective concentration range.

To sum up the experimental results, it can be seen that the active ingredients of *Salvia miltiorrhiza* Bunge of the present invention can be used for preparing an anti- SARS-CoV-2 drug, and the present invention has great clinical application value.

### BRIEF DESCRIPTION OF DRAWINGS

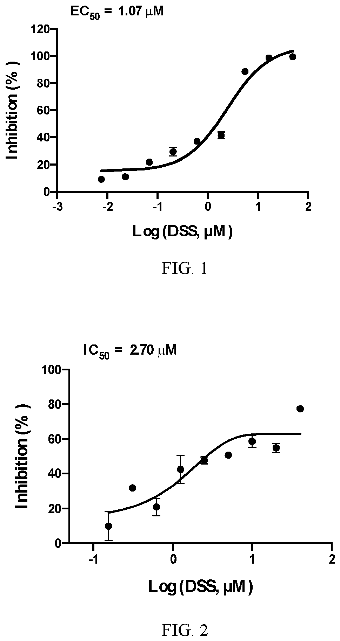

FIG. 1 shows an inhibition rate curve of inhibiting SARS-CoV-2 by salvianic acid A (DSS) at different concentrations in Embodiment 1 of the present invention, in which an abscissa represents a concentration of the salvianic acid A and an ordinate represents an inhibition rate of the salvianic acid A against the SARS-CoV-2 with a solvent group as a control, and a half effective concentration EC₅₀ value of the salvianic acid A to inhibit the SARS-CoV-2 is calculated according to the inhibition rate.
FIG. 2 shows an inhibition rate curve of inhibiting entering of SARS-CoV-2 pseudovirus into a target cell by salvianic acid A (DSS) at different concentrations in Embodiment 2 of the present invention, in which an abscissa represents a concentration of the salvianic acid A and an ordinate represents an inhibition rate of inhibiting the entering of the SARS-CoV-2 pseudovirus by the salvianic acid A with a solvent group as a control, and a half inhibitory concentration IC₅₀ value of the salvianic acid A to inhibit the entering of the SARS-CoV-2 pseudovirus is calculated.

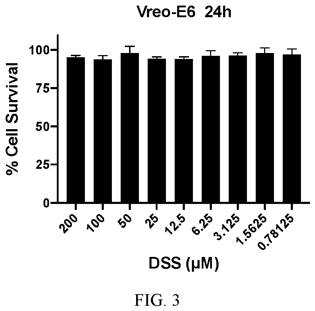

FIG. 3 shows a cell survival rate curve of a target cell Vero-E6 by salvianic acid A (DSS) in Embodiment 3 of the present invention, in which an abscissa represents a concentration of the salvianic acid A and an ordinate represents a percentage of cell survival of the cell Vero-E6 after administration of different concentrations of salvianic acid A with a solvent group as a control.

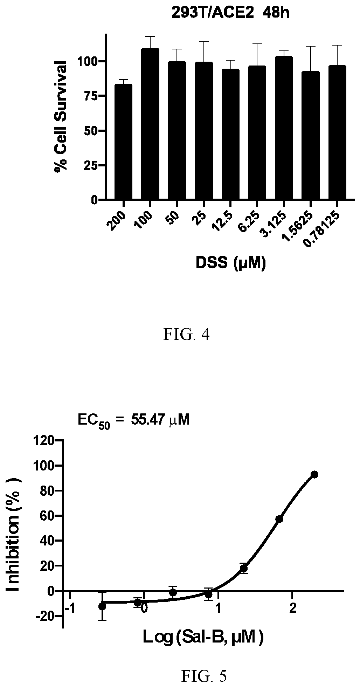

FIG. 4 shows a cell survival rate curve of a target cell 293T/ACE2 salvianic acid A (DSS) in Embodiment 3 of the present invention, in which an abscissa represents a concentration of the salvianic acid A and an ordinate represents a percentage of cell survival of the cell 293T/ACE2 after administration of different concentrations of salvianic acid A with a solvent group as a control.
FIG. 5 shows an inhibition rate curve of inhibiting SARS-CoV-2 by salvianolic acid B (Sal-B) at different concentrations in Embodiment 4 of the present invention, in which an abscissa represents a concentration of the Sal-B and an ordinate represents an inhibition rate of the Sal-B against the SARS-CoV-2 with a solvent group as a control, and a half effective concentration EC₅₀ value of the Sal-B to inhibit the SARS-CoV-2 is calculated according to the inhibition rate.

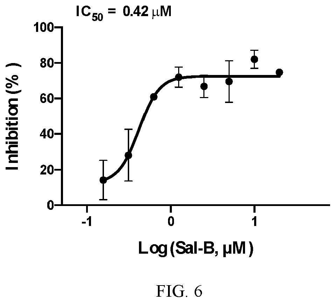

FIG. 6 shows an inhibition rate curve of inhibiting entering of SARS-CoV-2 pseudovirus into a target cell by salvianolic acid B (Sal-B) at different concentrations in Embodiment 5 of the present invention, in which an abscissa represents a concentration of the Sal-B and an ordinate represents an inhibition rate of inhibiting the entering of the SARS-CoV-2 pseudovirus by the Sal-B with a solvent group as a control, and a half inhibitory concentration IC₅₀ value of the Sal-B to inhibit the entering of the SARS-CoV-2 pseudovirus is calculated.

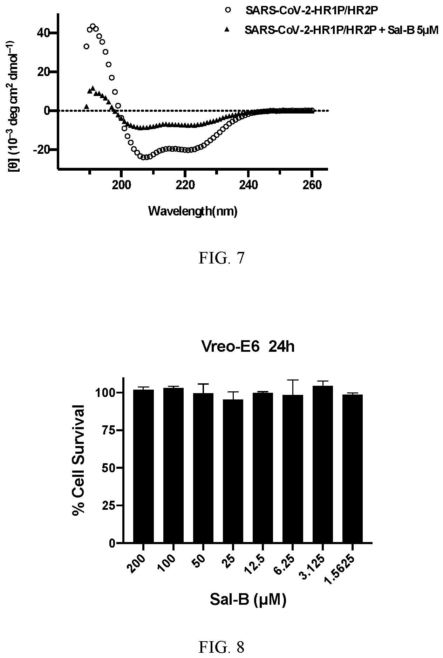

FIG. 7 is a curve schematic spectrogram of a scanning wavelength (abscissa) and a molar ratio of circumference to diameter (ordinate) of Circular Dichroism (CD) in Embodiment 6 of the present invention, with a scanning wavelength range of 190 nm to 260 nm. An α-helical configuration is formed spontaneously by six-helix core region polypeptides HR1P and HR2P of SARS-CoV-2 S protein, a spectrum shows double negative peaks at 208 nm and 222 nm (SARS-CoV-2-HR1P/HR2P), and a curve after administration of a standard concentration of 5 µM of salvianolic acid B is SARS-CoV-2-HR1P/HR2P + Sal-B 5µM.
FIG. 8 shows a cell survival rate curve of a target cell Vero-E6 by salvianolic acid B (Sal-B) in Embodiment 7 of the present invention, in which an abscissa represents a concentration of Sal-B and an ordinate represents a percentage of cell survival of the cell Vero-E6 after administration of different concentrations of Sal-B with a solvent group as a control.

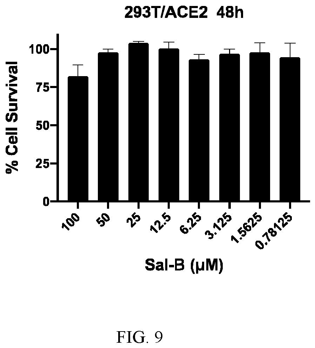

FIG. 9 shows a cell survival rate curve of a target cell 293T/ACE2 by the Sal-B in Embodiment 7 of the present invention, in which an abscissa represents a concentration of the Sal-B and an ordinate represents a percentage of cell survival of the cell 293T/ACE2 after administration of different concentrations of Sal-B with a solvent group as a control.

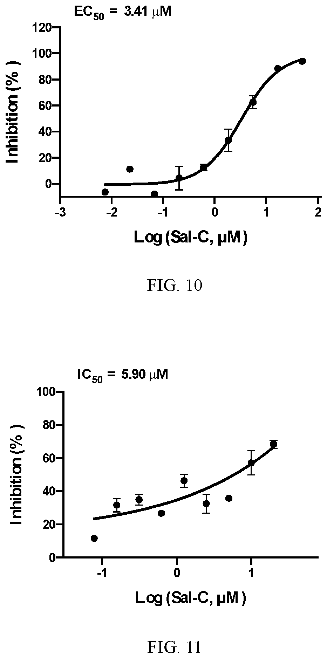

FIG. 10 shows an inhibition rate curve of inhibiting SARS-CoV-2 by salvianolic acid C (Sal-C) at different concentrations in Embodiment 8 of the present invention, in which an abscissa represents a concentration of the Sal-C and an ordinate represents an inhibition rate of the Sal-C against the SARS-CoV-2 with a solvent group as a control, and a half effective concentration EC₅₀ value of the Sal-C to inhibit the SARS-CoV-2 is calculated according to the inhibition rate.
FIG. 11 shows an inhibition rate curve of inhibiting entering of SARS-CoV-2 pseudovirus into a target cell by salvianolic acid C (Sal-C) at different concentrations in Embodiment 9 of the present invention, in which an abscissa represents a concentration of the Sal-C and an ordinate represents an inhibition rate of inhibiting the entering of the SARS-CoV-2 pseudovirus by the Sal-C with a solvent group as a control, and a half inhibitory concentration IC₅₀ value of the Sal-C to inhibit the entering of the SARS-CoV-2 pseudovirus is calculated.

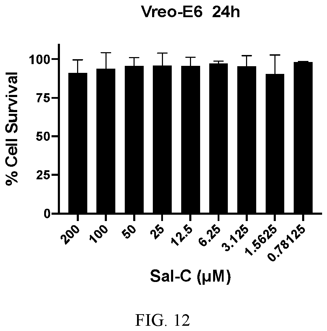

FIG. 12 shows a cell survival rate curve of a target cell Vero-E6 by salvianolic acid C (Sal-C) in Embodiment 10 of the present invention, in which an abscissa represents a concentration of the Sal-C and an ordinate represents a percentage of cell survival of the cell Vero-E6 after administration of different concentrations of Sal-C with a solvent group as a control.

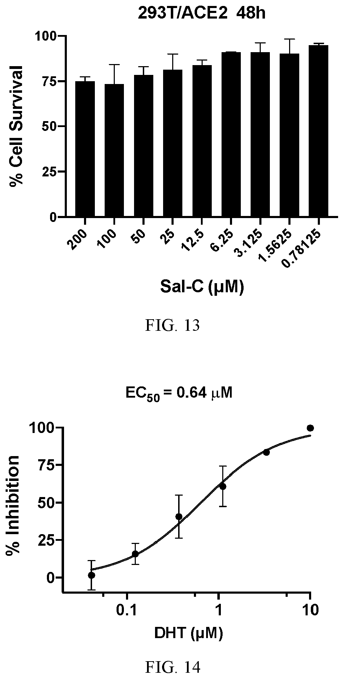

FIG. 13 shows a cell survival rate curve of a target cell 293T/ACE2 by the Sal-C in Embodiment 10 of the present invention, in which an abscissa represents a concentration of the Sal-C and an ordinate represents a percentage of cell survival of the cell 293T/ACE2 after administration of different concentrations of Sal-C with a solvent group as a control.
FIG. 14 shows an inhibition rate curve of inhibiting SARS-CoV-2 by concentration dependence of dihydrotanshinone I (DHT) in Embodiment 11 of the present invention, in which an abscissa represents a concentration of the DHT and an ordinate represents an inhibition rate of the DHT against the SARS-CoV-2 with a solvent group as a control, and a half effective concentration EC₅₀ value of the DHT to inhibit the SARS-CoV-2 is calculated according to the inhibition rate.

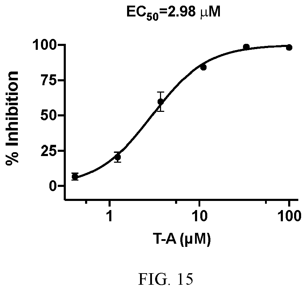

FIG. 15 shows an inhibition rate curve of inhibiting SARS-CoV-2 by Tanshinone I (T-A) at different concentrations in Embodiment 12 of the present invention, in which an abscissa represents a concentration of the T-A and an ordinate represents an inhibition rate of the T-A against the SARS-CoV-2 with a solvent group as a control, and a half effective concentration EC₅₀ value of the T-A to inhibit the SARS-CoV-2 is calculated according to the inhibition rate.

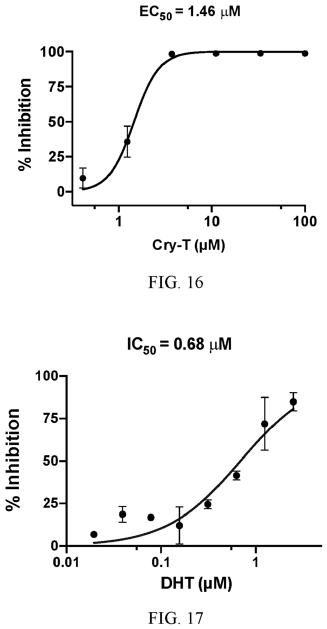

FIG. 16 shows an EC₅₀ value curve of inhibiting SARS-CoV-2 by Cryptotanshinone (Cry-T) in Embodiment 13 of the present invention, in which an abscissa represents a concentration of the Cry-T and an ordinate represents an inhibition rate of the Cry-T against the SARS-CoV-2 with a solvent group as a control, and a half effective concentration EC₅₀ value of the Cry-T to inhibit the SARS-CoV-2 is calculated according to the inhibition rate.
FIG. 17 shows an inhibition rate curve of inhibiting entering of a SARS-CoV-2 pseudovirus into a target cell by dihydrotanshinone I (DHT) at different concentrations in Embodiment 14 of the present invention, in which an abscissa represents a concentration of the DHT and an ordinate represents an inhibition rate of inhibiting the entering of the SARS-CoV-2 pseudovirus by the DHT with a solvent group as a control, and a half inhibitory concentration IC₅₀ value of the DHT to inhibit the entering of the SARS-CoV-2 pseudovirus is calculated.

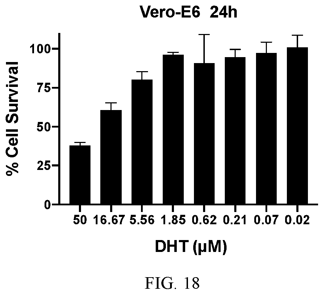

FIG. 18 shows a survival rate curve of a cell Vero-E6 by dihydrotanshinone I (DHT) in Embodiment 15 of the present invention, in which an abscissa represents a concentration of the DHT and an ordinate represents a percentage of cell survival of the cell Vero-E6 after administration of different concentrations of DHT with a solvent group as a control.

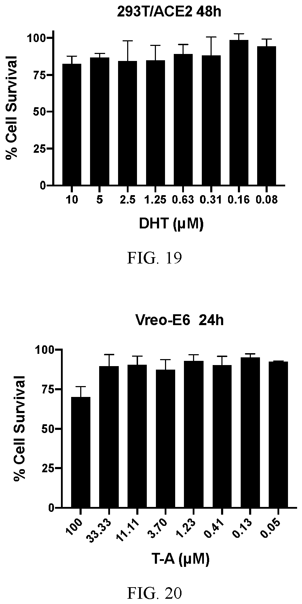

FIG. 19 shows a survival rate curve of a cell 293T/ACE2 by dihydrotanshinone I (DHT) in Embodiment 15 of the present invention, in which an abscissa represents a concentration of the DHT and an ordinate represents a percentage of cell survival of the cell 293T/ACE2 after administration of different concentrations of DHT with a solvent group as a control.
FIG. 20 shows a survival rate curve of a cell 293T/ACE2 by Tanshinone I (T-A) in Embodiment 15 of the present invention, in which an abscissa represents a concentration of the T-A and an ordinate represents a percentage of cell survival of the cell 293T/ACE2 after administration of different concentrations of T-A with a solvent group as a control.

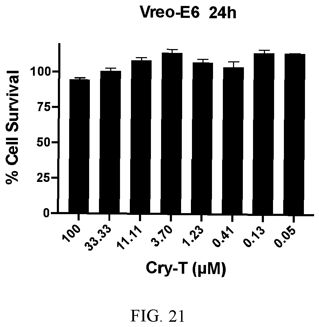

FIG. 21 shows a survival rate curve of a cell 293T/ACE2 by Cryptotanshinone (Cry-T) in Embodiment 15 of the present invention, in which an abscissa represents a concentration of the Cry-T and an ordinate represents a percentage of cell survival of the cell 293T/ACE2 after administration of different concentrations of Cry-T with a solvent group as a control.

### DETAILED DESCRIPTION OF EMBODIMENTS

In order to better understand the content of the present invention, the content of the present invention is further expounded below in combination with appended drawings and specific implementations, but the content of protection of the present invention is not limited to the following embodiments.

Unless defined otherwise, technical and scientific terms used herein have the same meaning as commonly understood by those skilled in the technical field of the present invention. The terms used in the specification of the present invention are for the purpose of describing specific embodiments, and are not limited to the present invention.

The following embodiments mainly evaluate the anti-SARS-CoV-2 infection activity of salvianic acid A, salvianolic acid B, salvianolic acid C, dihydrotanshinone I and analogues thereof by constructing an *in vitro* cell infection models of SARS-CoV-2 live virus and SARS-CoV-2 pseudovirus, and confirm that the salvianic acid A, the salvianolic acid B, the salvianolic acid C, the dihydrotanshinone I and the analogues thereof have the ability of anti-SARS-CoV-2 infection of target cells, and provide a use of the salvianic acid A, the salvianolic acid B, the salvianolic acid C, the dihydrotanshinone I and the analogues thereof in preparation of an anti-SARS-CoV-2 drug. In addition, it is also proved that inhibiting entering of SARS-CoV-2 into a target cell by the salvianolic acid B is inhibiting a change of a six-helix conformation of a subunit S2 of a SARS-CoV-2 S protein, thereby inhibiting virus-cell fusion mediated by the SARS-CoV-2 S protein.

Cells Vero-E6 and 293T used in the present invention are purchased from ATCC of the United States, and the cell 293T (293T/ACE2) stably overexpressing human SARS-CoV-2 receptor protein ACE2 is constructed and stored by the institution.

A cell growth culture solution used in embodiments of the present invention consists of a DMEM basic medium, in which fetal bovine serum of 10% of a total volume and ampicillin/streptomycin of 1% of a total volume are added, and the culture solution is stored at 4°C and is preheated by 37°C water bath before use.

Salvianic acid A used in the embodiments of the present invention is purchased from Shanghai TopScience Co., Ltd., with a purity of >99%.

Salvianolic acid B used in the embodiments of the present invention is purchased from Shanghai TopScience Co., Ltd., with a purity of >99%.

Salvianolic acid C used in the embodiments of the present invention is purchased from Shanghai TopScience Co., Ltd., with a purity of >99%.

Dihydrotanshinone I, Tanshinone I, Cryptotanshinone used in the embodiments of the present invention are purchased from Shanghai TopScience Co., Ltd., with a purity of >99%.

SARS-CoV-2 used in the embodiments of the present invention is isolated from a body of an infected person by Wuhan Institute of Virology, amplified and preserved.

In the embodiments of the present invention, a pseudovirus packaging plasmid and a source thereof are as follows: a pseudovirus packaging skeleton plasmid pNL4-3.Luc.R-E- is identified and preserved by Southern Medical University, and a disclosed optimized full-length SARS-CoV-2 S protein core plasmid pcDNA3.1-SARS-CoV-2-Sipke is presented by the professor L Lu of Shanghai Fudan University.

A single luciferase detection kit used in the embodiments of the present invention is purchased from PROMEGA Company of the United States, including a cell lysis solution and a luciferase reaction substrate.

Takara MiniBEST Viral RNA/DNA Extraction Kit, Takara PrimeScriptTM RT reagent Kit with gDNA Eraser, Takara TB Green^{®} Premix Ex Taq^{™} IITliRNaseH Plus used in the embodiments of the present invention are all purchased from Takara.

### Pharmacological Experiment

### Embodiment 1 Detection of inhibitory activity of salvianic acid A on SARS-CoV-2 in vitro

1. Inhibitory activity experiment of drug:
1) A cell Vero-E6 in a logarithmic growth period was taken, with 3^{∗}10^5 cells/well, inoculated into a 48-well plate, and cultured overnight at 37°C with 5% CO₂.
2) Drug pre-incubation: a DMEM medium containing fetal bovine serum of 2% of a total volume was used to dilute a drug. An initial concentration of the drug was set to 50 µM (a solvent was DMSO), the drug was diluted three times multiple proportions, and triplicate wells were set for each concentration, with a total of nine drug gradients (50, 16.7, 5.56, 1.85, 0.62, 0.21, 0.07, 0.023, 0.008 µM); a solvent dimethyl sulfoxide (DMSO) was set as a control group, and the control group used the DMEM medium containing the fetal bovine serum of 2% of the total volume for dilution, and the drug was administrated with the same volume of DMSO. After a cell supernatant was removed, 100 µL of a diluted drug was added into each well in an experimental group in the 48-well plate in 1), 100 µL of a diluted DMSO was added in the control group, and they were incubated at 37°C for 1 hour.
3) Virus infection: 5 µL of a SARS-CoV-2 virus diluent was added into each well of the 48-well plate (multiplicity of infection MOI=0.05), and continued to be incubated at 37°C for 1 hour. An infection supernatant was fully removed, and the cell was washed with 200 µL of PBS once. 200 µL of a corresponding concentration of medium containing the drug was re-added into the wells and continued to be cultured for 24 hours, and 150 µL of a cell culture supernatant was taken to be detected. The virus copy number was determined by qRT-PCR.
4) For a specific operation method of virus RNA extraction, referring to Takara MiniBEST Viral RNA/DNA Extraction Kit (Code No. 9766):
a. Virus lysis: 50 µL of a PBS solution (pH7.4) was added into 150 µL of the cell culture supernatant to supplement to 200 µL. 200 µL of a Buffer VGB, 20 µL of Proteinase K and 1.0 µL of Carrier RNA were added, fully mixed, and subjected to a water bath at 56°C for 10 minutes to be fully lysed. 200 µL of absolute ethyl alcohol was added into a lysis solution, and was fully sucked, beaten and mixed evenly.
b. Column chromatography: Spin Column was placed on Collection Tube, a solution was transferred into Spin Column, centrifuged at 12,000 rpm for 2 minutes, and a filtrate was discarded.
c. Washing 1: 500 µL of a Buffer RWA was added into the Spin Column, centrifuged at 12,000 rpm for 1 minute, and a filtrate was discarded.
d. Washing 2: 700 µL of a Buffer RWB was added into the Spin Column, centrifuged at 12,000 rpm for 1 minute, and a filtrate was discarded. (A specified volume of 100% ethyl alcohol has been added into the Buffer RWB). The Buffer RWB was added along a periphery of a tube wall of the Spin Column.
e. Step d was repeated.
f. Spin Column was placed on Collection Tube, and centrifuged at 12,000 rpm for 2 minutes.
g. Elution: Spin Column was placed on a new 1.5 mL RNase free collection tube, and 30 µL of RNase free dH₂O was added to a center of a Spin Column membrane, and stood at a room temperature for 5 minutes. Centrifugation was conducted at 12,000 rpm for 2 minutes for eluting RNA.

5) For a specific operation method of virus RNA reverse transcription, referring to Takara PrimeScriptTM RT reagent Kit with gDNA Eraser (Code No. RR047A):
a. Removing genomic DNA from an eluate: a reaction system was prepared on ice according to the following components.

| **Reagents** | **Volume (µL)** |
|---|---|
| 5^{∗}gDNA Eraser Buffer | 2.0 |
| gDNA Eraser | 1.0 |
| Total RNA | 3.0 |
| RNase Free dHzO | 4.0 |
| Total volume | 10.0 |

A sample was placed at 42°C and reacted for 2 minutes.
b. Reverse transcription reaction:

| **Reagents** | **Volume (µL)** |
|---|---|
| A reaction solution in step 1 | 10.0 |
| PrimeScript RT Enzyme Mix I | 1.0 |
| RT Primer Mix | 1.0 |
| 5×PrimeScript Buffer 2 (for Real Time) | 4.0 |
| RNase Free dH₂O | Supplement to 20.0 |

The sample was incubated at 37°C for 15 minutes and then heated at 85°C for 5 seconds.
6) Detection of the virus copy number by qPCR: referring to Takara TB Green^{®} Premix Ex Taq^{™} II (TliRNaseH Plus, Code, No. RR820A) (using a standard curve method: an RBD plasmid with known copy number was used as a standard, and a specific primer was targeted to RBD). A reaction system was prepared on ice according to the following components.

| **Reagents** | **Volume (µL)** |
|---|---|
| TB Green Premix Ex Taq II (Tli RNaseH Plus) (2×) | 10 |
| Forward Primer (10 µM) | 1 |
| Reverse Primer (10 µM) | 1 |
| ROX Reference Dye (50X) | 0.4 |
| cDNA template | 1 |
| Sterile water | 6.6 |
| Total volume | 20 |

A primer sequence was as follows:
RBD Forward Primer: CAATGGTTTAACAGGCACAGG (SEQ ID NO:1)
RBD Reverse Primer: CTCAAGTGTCTGTGGATCACG (SEQ ID NO:2)
Computer detection: ABI7500 quantitative PCR instrument
Initial denaturation: 95°C, 30 seconds, 1 cycle; PCR amplification: 95°C, 5 seconds, 40 cycles; annealing: 60°C, 30 to 34 seconds; recording.

2. Results: as shown in FIG. 1;

The copy number of each sample was calculated according to a standard curve. An inhibition rate in the drug treatment group was calculated with the copy number in the DMSO group as reference. Then, according to the inhibition rates of different concentrations of the drug treatment group, Prism8.0 software was used to fit a drug inhibition rate curve, and a half effective concentration EC₅₀ of Salvianic acid A (DSS) on activity of SARS-CoV-2 was calculated to be 1.07 µM.

### Embodiment 2 Detection of inhibitory activity of Salvianic acid A on entering of SARS-CoV-2 pseudovirus

1. Method:
1) SARS-CoV-2 pseudovirus packaging:
A cell HEK-293T in a logarithmic growth period was taken, with 4^{∗}10^5 cells/mL, 2 mL each well thereof was evenly inoculated in a 6-well plate. Culture was conducted in a 37°C cell incubator with 5% CO₂ for 24 hours. A medium was changed with a fresh medium half an hour before transfection. 100 µL of a blank DMEM medium is used to prepare a plasmid diluent and a transfection reagent (PolyJet) diluent respectively, and a preparation proportion of each well was as follows (plasmid DNA needed to be extracted with an endotoxin-free extraction kit):

| | |
|---|---|
| pNL4-3.Luc.R-E- | 1000 ng |
| pcDNA3.1-SARS-CoV-2-Sipke | 500 ng |
| PolyJet | 6 µL |

A specific preparation method was as follows: a pNL4-3.Luc.R-E-plasmid and a pcDNA3.1-SARS-CoV-2-Sipke plasmid were added into 100 µL of a blank DMEM medium at the same time to be mixed evenly, and Poly Jet was diluted and mixed evenly with 100 µL of a blank DMEM medium. The PolyJet diluent was added into the plasmid diluent and mixed evenly, incubated at a room temperature for 10 minutes, and added evenly into the cell HEK-293T. After culture at 37°C for 48 hours, a supernatant virus solution was collected, which was centrifuged at 4,000 rpm for 10 minutes and filtered with a 0.45 µM sterile filter head, and a SARS-CoV-2 pseudovirus was obtained.
2) Pseudovirus inhibition experiment:
A cell 293T (293T/ACE2) overexpressing a SARS-CoV-2 receptor ACE2 in a logarithmic growth period was taken and evenly spread in a 96-well cell plate according to 1^{∗}10^4 cells/well. Culture was conducted in a 37°C cell incubator for 24 hours.

An initial concentration of a drug was set to 40 µM, and was diluted to nine concentration gradients, respectively 40, 20, 10, 5, 2.5, 1.25, 0.625, 0.3125, 0.15625 µM, with a DMEM medium containing fetal bovine serum of 2% of a total volume in twice multiple proportions. A DMSO solvent control group was set with a volume of 60 µL per well and triplicate wells for each concentration. 60 µL of a pseudovirus solution was added into a diluted drug and acted at a room temperature for 30 minutes, and 100 µL/well thereof was taken to be added into the cell ACE2/293T and cultured at 37°C for 48 hours. The medium was removed. The cell was washed with 100 µL/well of sterile PBS (pH7.4) once. 50 µL of a 1X cell lysis solution was added into each well, and lysed under oscillation at a room temperature for 15 minutes. 40 µL/well of a lysis supernatant was transferred into a 96-well white Enzyme Linked Immunosorbent Assay (ELISA) plate. An equal volume of a diluted luciferase substrate was added according to the instructions of a single luciferase detection kit, a fluorescence value was immediately detected by a microplate reader, and according to the fluorescence value, the activity of Salvianic acid A to inhibit absorption and entering of the virus was judged. An inhibition rate was calculated according to a corresponding relationship between the fluorescence value and a drug concentration. A curve was drawn, and a half inhibitory concentration IC₅₀ of Salvianic acid A was calculated.

2. Results: as shown in FIG. 2;

An inhibition rate in the drug treatment group was calculated according to the fluorescence value with the DMSO group as a control. Then, according to the inhibition rates of different concentrations of the drug treatment group, Prism8.0 software was used to fit a drug inhibition rate curve, and a half inhibitory concentration IC₅₀ of salvianic acid A (DSS) to inhibit entering of the SARS-CoV-2 pseudovirus into a target cell was calculated to be 2.70 µM.

### Embodiment 3 Cytotoxicity detection of Salvianic acid A

1. Method:
   1) Cell inoculation:
      Cells Vero-E6 and 293T/ACE2 in a logarithmic growth period were taken, cell densities thereof were adjusted to 1^{∗}10^4 cells/well, and 100 µL/well thereof were respectively inoculated on a 96-well plate and cultured overnight.
   2) Drug concentration design:
      Before administration, a drug was diluted to nine concentration gradients with a DMEM medium containing fetal bovine serum of 2% of a total volume twice multiple proportions, and an initial concentration of the drug was set to 200 µM (200, 100, 50, 25, 12.5, 6.25, 3.125, 1.5625, 0.78125 µM), and 100 µL per well of a diluted drug was taken to be added into the cells Vero-E6 and 293T/ACE2 in the 96-well plate in 1), with a final volume of 200 µL per well. Triplicate wells were set for each drug concentration. A DMSO solvent treatment group was used as a blank control.
   3) Absorbance detection:
      After incubation in an incubator for 48 hours, 10 µL of a CCK-8 working solution was added into each well, and incubation continued to be conducted in the incubator for 3 hours. The absorbance at 450 nm was measured by a microplate reader.
   4) According to measured OD values, survival rates of the cells Vero-E6 and 293T/ACE2 under the action of various concentrations of the drug were calculated respectively compared with the control group.
2. Results: as shown in FIG. 3 and FIG. 4;
   salvianic acid A (DSS) had no obvious toxic effect on the cell Vero-E6 (FIG. 3) and the cell 293T/ACE2 (FIG. 4) at 200 µM and within an effective concentration range.

### Embodiment 4 Detection of inhibitory activity of salvianolic acid B on SARS-CoV-2 in vitro

1. Method:
1) A cell Vero-E6 in a logarithmic growth period was inoculated into a 48-well plate, with 3^{∗}10^5 cells/well, and cultured overnight at 37°C with 5% CO₂.
2) Drug pre-incubation: a DMEM medium containing fetal bovine serum of 2% of a total volume was used to dilute a drug. An initial concentration of salvianolic acid B was set to 200 µM (a solvent was DMSO), the drug was diluted three times multiple proportions, and triplicate wells were set for each concentration of drug, with a total of seven drug gradients (200, 66.67, 22.22, 7.41, 2.47, 0.82, 0.27 µM); a solvent dimethyl sulfoxide (DMSO) was set as a control group, and the control group used the DMEM medium containing the fetal bovine serum of 2% of the total volume for dilution, and the drug was administrated with the same volume of DMSO. After a cell supernatant was removed, 100 µL of a diluted drug was added into each well in an experimental group in the 48-well plate in 1), 100 µL of a diluted DMSO was added in the control group, and they were incubated at 37°C for 1 hour.
3) Virus infection: 5 µL of a SARS-CoV-2 virus diluent was added into each well of the 48-well plate (multiplicity of infection MOI=0.05), and the cell was placed at 37°C to continue to be incubated for 1 hour.
4) Solution change: An infection supernatant was fully removed, and the cell was washed with 200 µL of PBS once. 200 µL of a medium containing the drug with a corresponding concentration was re-added into each well and continued to be cultured at 37°C for 24 hours, and 150 µL of a cell culture supernatant was then taken to be detected. The virus copy number in the supernatant was determined by qRT-PCR to evaluate the anti-SARS-CoV-2 virus activity of the drug.
5) For a specific operation method of virus RNA extraction, referring to Takara MiniBEST Viral RNA/DNA Extraction Kit (Code No. 9766):
a) Virus lysis: 50 µL of a PBS solution (pH7.4) was added into 150 µL of the cell culture supernatant to supplement to 200 µL. Then, 200 µL of a Buffer VGB, 20 µL of Proteinase K and 1.0 µL of Carrier RNA were added, fully mixed, and subjected to warm bath in a 56°C water bath for 10 minutes to be fully lysed. 200 µL of absolute ethyl alcohol was added into a lysis solution, and was fully sucked, beaten and mixed evenly.
b) Spin Column was placed on Collection Tube, a solution was transferred into Spin Column, centrifuged at 12,000 rpm for 2 minutes, and a filtrate was discarded.
c) 500 µL of a Buffer RWA was added into the Spin Column, centrifuged at 12,000 rpm for 1 minute, and a filtrate was discarded.
d) 700 µL of a Buffer RWB was added into the Spin Column, centrifuged at 12,000 rpm for 1 minute, and a filtrate was discarded. (A specified volume of 100% ethyl alcohol has been added into the Buffer RWB). The Buffer RWB was added along a periphery of a tube wall of the Spin Column, which was helpful to completely flush salt adhering to the tube wall.
e) Step d was repeated.
f) Spin Column was placed on Collection Tube, and centrifuged at 12,000 rpm for 2 minutes.
g) Spin Column was placed on a new 1.5 mL RNase free collection tube, and 30 µL of RNase free dH₂O was added to a center of a Spin Column membrane, and stood at a room temperature for 5 minutes. Centrifugation was conducted at 12,000 rpm for 2 minutes for eluting RNA.

6) A specific operation method of virus RNA reverse transcription (referring to Takara PrimeScriptTM RT reagent Kit with gDNA Eraser, Code No. RR047A):
a) Reaction for removing genomic DNA: a reaction mixed solution was prepared on ice according to the following components.

| **Reagents** | **Volume (µL)** |
|---|---|
| 5^{∗}gDNA Eraser Buffer | 2.0 |
| gDNA Eraser | 1.0 |
| Total RNA | 3.0 |
| RNase Free dH₂O | 4.0 |
| Total volume | 10.0 |

A sample was placed at 42°C and reacted for 2 minutes.
b) Reverse transcription reaction: preparation on ice

| **Reagents** | **Volume (µL)** |
|---|---|
| A reaction solution in step 1 | 10.0 |
| PrimeScript RT Enzyme Mix I | 1.0 |
| RT Primer Mix | 1.0 |
| 5×PrimeScript Buffer 2 (for Real Time) | 4.0 |
| RNase Free dH₂O | Supplement to 20.0 |

The sample was incubated at 37°C for 15 minutes and then heated at 85°C for 5 seconds.
7) Detection of the virus copy number by qPCR: referring to Takara TB Green^{®} Premix Ex Taq^{™} II (TliRNaseH Plus, Code No. RR820A) (using a standard curve method: an RBD plasmid with known copy number was used as a standard, and a specific primer was targeted to RBD). A reaction solution was prepared on ice according to the following components.

| **Reagents** | Usage (µL) |
|---|---|
| TB Green Premix Ex Taq II (Tli RNaseH Plus) (2X) | 10 |
| Forward Primer (10 µM) | 1 |
| Reverse Primer (10 µM) | 1 |
| ROX Reference Dye (50X) | 0.4 |
| cDNA template | 1 |
| Sterile water | 6.6 |
| Total volume | 20 |

A primer sequence was as follows:
RBD Forward Primer: CAATGGTTTAACAGGCACAGG (SEQ ID NO:1)
RBD Reverse Primer: CTCAAGTGTCTGTGGATCACG (SEQ ID NO:2)
According to a two-step PCR amplification standard procedure, detection was completed on an ABI7500 quantitative PCR instrument:
   Stage 1: initial denaturation, Reps: 1 cycle, 95°C, 30 seconds;
   Stage 2: PCR reaction, Reps: 40 cycles, 95°C, 5 seconds;
   Annealing: 60°C, 30 to 34 seconds.

2. Results: as shown in FIG. 5;

The copy number of each sample was calculated according to a standard curve. An inhibition rate in the drug treatment group was calculated with the copy number in the DMSO group as reference. Then, according to the inhibition rates of different concentrations of the drug treatment group, Prism8.0 software was used to fit a drug inhibition rate curve, and a half effective concentration EC₅₀ of salvianolic acid B (Sal-B) resisting activity of SARS-CoV-2 was calculated to be 55.47 µM.

### Embodiment 5 Detection of inhibitory activity of salvianolic acid B on entering of SARS-CoV-2 pseudovirus

1. Method:
1) SARS-CoV-2 (pNL4-3.Luc.R-E-pcDNA3.1-SARS-CoV-2-Sipke) pseudovirus packaging:
A cell HEK-293T in a logarithmic growth period was taken, with 4^{∗}10^5 cells/mL, 2 mL each well thereof was evenly inoculated in a 6-well plate. Culture was conducted in a 37°C cell incubator with 5% CO₂ for 24 hours. A medium was changed with a fresh medium 1 hour before transfection. 100 µL of a blank DMEM medium is used to prepare a plasmid diluent and a transfection reagent (PolyJet) diluent respectively, and a preparation proportion of each well was as follows (plasmid DNA needed to be extracted with an endotoxin-free extraction kit):

| | |
|---|---|
| pNL4-3.Luc.R-E- | 1000 ng |
| pcDNA3.1-SARS-CoV-2-Sipke | 500 ng |
| PolyJet | 6 µL |

A specific preparation method was as follows: a pNL4-3.Luc.R-E-plasmid and a pcDNA3.1-SARS-CoV-2-Sipke plasmid were added into 100 µL of a blank DMEM medium at the same time to be mixed evenly, and Poly Jet was diluted and mixed evenly with 100 µL of a blank DMEM medium. The PolyJet diluent was added into the plasmid diluent and mixed evenly, incubated at a room temperature for 15 minutes, and added evenly into the cell HEK-293T. After culture at 37°C for 48 hours, a supernatant virus solution was collected, which was centrifuged at 4,000 rpm for 10 minutes and filtered with a 0.45 µM sterile filter head, and a SARS-CoV-2 pseudovirus was obtained.
2) Pseudovirus inhibition experiment:
Effects of a drug and the pseudovirus: A cell 293T (293T/ACE2) overexpressing a SARS-CoV-2 receptor ACE2 in a logarithmic growth period was taken and evenly spread in a 96-well cell plate according to 1^{∗}10^4 cells/well. Culture was conducted in a 37°C cell incubator for 24 hours.

An initial concentration of salvianolic acid B was set to 20 µM, which was, before administration, diluted to eight concentration gradients (20, 10, 5, 2.5, 1.25, 0.625, 0.3125, 0.15625 µM) with a DMEM medium containing fetal bovine serum of 2% of a total volume in twice multiple proportions. A DMSO solvent control group was set with 60 µL per well and triplicate wells for each concentration. 60 µL of the pseudovirus was added into a diluted drug and mixed evenly for acting at a room temperature for 30 minutes, and 100 µL/well thereof was taken to be added into the cell ACE2/293T and cultured at 37°C for 48 hours.

Detection: the medium was removed. The cell was washed with 200 µL/well of sterile PBS (pH7.4) once. 40 µL of a 1X cell lysis solution was added into each well, and lysed under oscillation at a room temperature for 15 minutes. 30 µL/well of a lysis supernatant was transferred into a 96-well white ELISA plate. An equal volume of a diluted luciferase substrate was added according to the instructions of a single luciferase detection kit, a fluorescence value was immediately detected by a microplate reader, and according to the fluorescence value, the activity of salvianic acid A to inhibit absorption and entering of the virus was judged. An inhibition rate was calculated according to a corresponding relationship between the fluorescence value and a drug concentration. A curve was drawn, and a half inhibitory concentration IC₅₀ of salvianolic acid B was calculated.

2. Results: as shown in FIG. 6;
An inhibition rate in the drug treatment group was calculated according to the fluorescence value with the DMSO group as a control. Then, according to the inhibition rates of different concentrations of the drug treatment group, Prism8.0 software was used to fit a drug inhibition rate curve, and a half inhibitory concentration IC₅₀ of salvianolic acid B (Sal-B) to inhibit entering of the SARS-CoV-2 pseudovirus into a target cell was calculated to be 0.416 µM.

### Embodiment 6 Inhibition of salvianolic acid B on formation of six-helix (6-HB) by SARS-CoV-2 HR1P and HR2P: interfering α-helical conformation

During SARS-CoV-2 infection, HR1 and HR2 of a subunit S2 of an SARS-CoV-2 S protein can spontaneously polymerize to form a six-helix (6-HB) structure with an α-helical conformation, thereby promoting membrane fusion of a virus and a target cell, further promoting the virus to enter the cell. We synthesized SARS-CoV-2 HR1P and SARS-CoV-2 HR2P polypeptides located in a core region of the 6-HB in vitro to observe whether salvianolic acid B acts on the 6-HB.

An amino acid sequence of SARS-CoV-2 HR1P is as follows:
ANQFNSAIGKIQDSLSSTASALGKLQDVVNQNAQALNTLVKQ (SEQ ID NO:3)

An amino acid sequence of SARS-CoV-2-HR2P is as follows:
DISGINASVVNIQKEIDRLNEVAKNLNESLIDLQEL (SEQ ID NO:4)

Circular dichroism (CD) is used to analyze a secondary conformation (α-helix, β-fold or random coil) of a protein by using different absorbances of asymmetric molecules to left and right circularly polarized light. A CD spectrum value of the α-helix has two negative peaks at 208 nm and 222 nm, and 100% α-helix shows a -33000 absorption peak at 222 nm of CD spectrum, so a proportion of an α-helix structure in one protein molecule can be calculated by Xα = (-[θ]₂₂₂) /-33000.
1. Method:
   1) Sample preparation:
      HR1P and HR2P polypeptides were prepared into 10 µM of a working solution with a 50 mM, pH 7.2 PBS solution.
      System preparation: 0.15 µL of salvianolic acid B with a standard concentration of 5 mM was added into 150 µL of HR1P (10 µM) and mixed evenly, in a control group, 150 µL of HR1P (10 µM) and 0.15 µL of DMSO were mixed and acted at a room temperature for 30 minutes, 150 µL of HR2P (10 µM) was added, and incubation continued to be conducted at a room temperature for 30 minutes.
   2) Circular dichroism spectrometer parameter setting (static spectrum):
      Detecting a temperature: 4°C, scanning a wavelength range: 190-260 nm, a bandwidth: 2 nm, a step: 1 nm, time of each point: 0.5 second.
   3) Sample detection:
      A sample was added into a 1 mm optical path cuvette, CD wavelength scanning was conducted, a CD signal value [θ] was saved, a CD curve was drawn, and an α-helix percentage was calculated according to a value [θ]₂₂₂.

### 2. Results: as shown in FIG. 7;

The α-helix was formed after SARS-CoV-2 HR1P and HR2P were combined, and a CD spectrum value (ordinate) thereof had two negative peaks at 208 nm and 222 nm. A percentage of an α-helix by combination by 10 µM of HR1P and HR2P was 60.67%, and after administration of 5 µM of salvianolic acid B (Sal-B), a percentage of an α-helix by combination by HR1P and HR2P decreased to 22.24%.

### Embodiment 7 Cytotoxicity detection of salvianolic acid B

1. Method:
   1) Cell inoculation:
      Cells Vero-E6 and 293T/ACE2 in a logarithmic growth period were taken, cell densities thereof were adjusted to 1^{∗}10^4 cells/well, and 100 µL/well thereof were respectively inoculated on a 96-well plate and cultured overnight in a 37°C cell incubator.
   2) Drug concentration design:
      Before administration, a drug was diluted to eight concentration gradients with a DMEM medium containing fetal bovine serum of 2% of a total volume twice multiple proportions.
      Cell Vero-E6: an initial concentration was set to 200 µM (200, 100, 50, 25, 12.5, 6.25, 3.125, 1.5625 µM), and 100 µL per well of a diluted drug was taken to be added into the cell Vero-E6 in the 96-well plate in 1), with a final volume of 200 µL per well. Triplicate wells were set for each drug concentration. A DMSO solvent treatment group was used as a blank control.
      Cell 293T/ACE2: an initial concentration was set to 100 µM (100, 50, 25, 12.5, 6.25, 3.125, 1.5625, 0.78125 µM), and 100 µL per well of a diluted drug was taken to be added into the cell 293T/ACE2 in the 96-well plate in 1), with a final volume of 200 µL per well. Triplicate wells were set for each drug concentration. A DMSO solvent treatment group was used as a blank control.
   3) Absorbance detection:
      After incubation in an incubator for 48 hours, 10 µL of a CCK-8 working solution was added into each well, and incubation continued to be conducted in the incubator for 3 hours. The absorbance at 450 nm was measured by a microplate reader.
   4) According to measured OD values, survival rates of the cells Vero-E6 and 293T/ACE2 under the action of various concentrations of the drug were calculated respectively compared with the control group.

### 2. Results: as shown in FIG. 8 and FIG. 9;

Salvianolic acid B (Sal-B) had no obvious toxic effect on the cell Vero-E6 at 200 µM and within an effective concentration range (FIG. 8). The Sal-B had no obvious toxic effect on the cell 293T/ACE2 (FIG. 9) at 100 µM and within an effective concentration range.

### Embodiment 8 Detection of inhibitory activity of salvianolic acid C on SARS-CoV-2 in vitro

1. Determination of inhibitory activity of drug on SARS-CoV-2
1) A cell Vero-E6 in a logarithmic growth period was inoculated into a 48-well plate, with 3^{∗}10^5 cells/well, and cultured overnight at 37°C with 5% CO₂.
2) Drug pre-incubation: a DMEM medium containing fetal bovine serum of 2% of a total volume was used to dilute a drug. An initial concentration of the drug was set to 50 µM (a solvent was DMSO), the drug was diluted three times multiple proportions, and triplicate wells were set for each concentration of the drug, with a total of nine drug gradients (50, 16.7, 5.56, 1.85, 0.62, 0.21, 0.07, 0.023, 0.008 µM); a solvent dimethyl sulfoxide (DMSO) was set as a control group, and the control group used the DMEM medium containing the fetal bovine serum of 2% of the total volume for dilution, and the drug was administrated with the same volume of DMSO. After a cell supernatant was removed, 100 µL of a diluted drug was added into each well in an experimental group in the 48-well plate in 1), 100 µL of a diluted DMSO was added in the control group, and they were incubated at 37°C for 1 hour.
3) Virus infection: 5 µL of a SARS-CoV-2 virus diluent was added into each well of the 48-well plate (multiplicity of infection MOI=0.05), and continued to be incubated at 37°C for 1 hour.
4) Solution change: An infection supernatant was fully removed, and the cell was washed with 200 µL of PBS once. 200 µL of a corresponding concentration of medium containing the drug was re-added into the wells and continued to be cultured for 24 hours, and 150 µL of a cell culture supernatant was taken to be detected. The virus copy number was determined by qRT-PCR.
5) A specific operation method of virus RNA extraction (referring to Takara MiniBEST Viral RNA/DNA Extraction Kit Code No. 9766):
a) Virus lysis: 50 µL of a PBS solution (pH7.4) was added into 150 µL of the cell culture supernatant to supplement to 200 µL. Then, 200 µL of a Buffer VGB, 20 µL of Proteinase K and 1.0 µL of Carrier RNA were added, fully mixed, and subjected to warm bath in a 56°C water bath for 10 minutes to be fully lysed. 200 µL of absolute ethyl alcohol was added into a lysis solution, and was fully sucked, beaten and mixed evenly.
b) Spin Column was placed on Collection Tube, a solution was transferred into Spin Column, centrifuged at 12,000 rpm for 2 minutes, and a filtrate was discarded.
c) 500 µL of a Buffer RWA was added into the Spin Column, centrifuged at 12,000 rpm for 1 minute, and a filtrate was discarded.
d) 700 µL of a Buffer RWB was added into the Spin Column, centrifuged at 12,000 rpm for 1 minute, and a filtrate was discarded. (A specified volume of 100% ethyl alcohol has been added into the Buffer RWB). The Buffer RWB was added along a periphery of a tube wall of the Spin Column, which was helpful to completely flush salt adhering to the tube wall.
e) Step d was repeated.
f) Spin Column was placed on Collection Tube, and centrifuged at 12,000 rpm for 2 minutes.
g) Spin Column was placed on a new 1.5 mL RNase free collection tube, and 30 µL of RNase free dH₂O was added to a center of a Spin Column membrane, and stood at a room temperature for 5 minutes. Centrifugation was conducted at 12,000 rpm for 2 minutes for eluting RNA.

6) A specific operation method of virus RNA reverse transcription (referring to Takara PrimeScriptTM RT reagent Kit with gDNA EraserCode No.RR047A):
a) Reaction for removing genomic DNA: a reaction mixed solution was prepared on ice according to the following components.

| **Reagents** | **Volume (µL)** |
|---|---|
| 5^{∗}gDNA Eraser Buffer | 2.0 |
| gDNA Eraser | 1.0 |
| Total RNA | 3.0 |
| RNase Free dH₂O | 4.0 |
| Total volume | 10.0 |

Reaction procedure: 42°C, 2 minutes.
b) Reverse transcription reaction: preparation on ice

| **Reagents** | **Volume (µL)** |
|---|---|
| A reaction solution in step 1 | 10.0 |
| PrimeScript RT Enzyme Mix I | 1.0 |
| RT Primer Mix | 1.0 |
| 5×PrimeScript Buffer 2 (for Real Time) | 4.0 |
| RNase Free dH₂O | Supplement to 20.0 |

Reaction procedure: 37°C, 15 minutes; 85°C, 5 seconds.
7) Detection of the virus copy number by qPCR: referring to Takara TB Green^{®} Premix Ex Taq^{™} II (TliRNaseH Plus, Code No. RR820A) (using a standard curve method: an RBD plasmid with known copy number was used as a standard, and a specific primer was targeted to RBD). A reaction solution was prepared on ice according to the following components.

| **Reagents** | **Volume (µL)** |
|---|---|
| TB Green Premix Ex Taq II (Tli RNaseH Plus) (2X) | 10 |
| Forward Primer (10 µM) | 1 |
| Reverse Primer (10 µM) | 1 |
| ROX Reference Dye (50X) | 0.4 |
| cDNA template | 1 |
| Sterile water | 6.6 |
| Total volume | 20 |

A primer sequence was as follows:
RBD Forward Primer: CAATGGTTTAACAGGCACAGG (SEQ ID NO:1)
RBD Reverse Primer: CTCAAGTGTCTGTGGATCACG (SEQ ID NO:2)
Detection was completed on an ABI7500 quantitative PCR instrument:
   Stage 1: initial denaturation, Reps: 1 cycle, 95°C, 30 seconds;
   Stage 2: PCR reaction, Reps: 40 cycles, 95°C, 5 seconds;
   Annealing: 60°C, 30 to 34 seconds.

2. Results: as shown in FIG. 10;
The copy number of each sample was calculated according to a standard curve. An inhibition rate in the drug treatment group was calculated with the copy number in the DMSO group as reference. Then, according to the inhibition rates of different concentrations of the drug treatment group, Prism8.0 software was used to fit a drug inhibition rate curve, and a half effective concentration EC₅₀ of salvianolic acid C (Sal-C) on SARS-CoV-2 was calculated to be 3.41 µM.

### Embodiment 9 Detection of inhibitory activity of salvianolic acid C on entering of SARS-CoV-2 pseudovirus

1. Method:
1) SARS-CoV-2 pseudovirus packaging:
A cell HEK-293T in a logarithmic growth period was taken, with 4^{∗}10^5 cells/mL, 2 mL each well thereof was evenly inoculated in a 6-well plate. Culture was conducted in a 37°C cell incubator with 5% CO₂ for 24 hours. A medium was changed with a fresh medium 1 hour before transfection. 100 µL of a blank DMEM medium is used to prepare a plasmid diluent and a transfection reagent (PolyJet) diluent respectively, and a preparation proportion of each well was as follows (plasmid DNA needed to be extracted with an endotoxin-free extraction kit):

| | |
|---|---|
| pNL4-3.Luc.R-E- | 1000 ng |
| pcDNA3.1-SARS-CoV-2-Sipke | 500 ng |
| PolyJet | 6 µL |

A specific preparation method was as follows: a pNL4-3.Luc.R-E-plasmid and a pcDNA3.1-SARS-CoV-2-Sipke plasmid were added into 100 µL of a blank DMEM medium at the same time to be mixed evenly, and Poly Jet was diluted and mixed evenly with 100 µL of a blank DMEM medium. The PolyJet diluent was added into the plasmid diluent and mixed evenly, incubated at a room temperature for 15 minutes, and added evenly into the cell HEK-293T. After culture at 37°C for 48 hours, a supernatant virus solution was collected, which was centrifuged at 4,000 rpm for 10 minutes and filtered with a 0.45 µM sterile filter head, and a SARS-CoV-2 pseudovirus was obtained.
2) Pseudovirus inhibition experiment:
A cell 293T (293T/ACE2) overexpressing a SARS-CoV-2 receptor ACE2 in a logarithmic growth period was taken and evenly spread in a 96-well cell plate according to 1^{∗}10^4 cells/well. Culture was conducted in a 37°C cell incubator for 24 hours.

An initial concentration of a drug was set to 20 µM, which was, before administration, diluted to nine concentration gradients (20, 10, 5, 2.5, 1.25, 0.625, 0.3125, 0.15625, 0.07813 µM) with a DMEM medium containing fetal bovine serum of 2% of a total volume twice multiple proportions. A DMSO solvent control group was set with 60 µL per well and triplicate wells for each concentration. 60 µL of the pseudovirus was added into a diluted drug and mixed evenly for acting at a room temperature for 30 minutes, and 100 µL/well thereof was taken to be added into the cell ACE2/293T and cultured at 37°C for 48 hours. The medium was removed. The cell was washed with 100 µL/well of sterile PBS (pH7.4) once. 50 µL of a 1X cell lysis solution was added into each well, and lysed under oscillation at a room temperature for 15 minutes. 40 µL/well of a lysis supernatant was transferred into a 96-well white ELISA plate. An equal volume of a diluted luciferase substrate was added according to the instructions of a single luciferase detection kit, a fluorescence value was immediately detected by a microplate reader, and according to the fluorescence value, the activity of salvianic acid A to inhibit absorption and entering of the virus was judged. An inhibition rate was calculated according to a corresponding relationship between the fluorescence value and a drug concentration. A curve was drawn and a half inhibitory concentration IC₅₀ of the drug was calculated.

2. Results: as shown in FIG. 11;
An inhibition rate in the drug treatment group was calculated according to the fluorescence value with the DMSO group as a control. Then, according to the inhibition rates of different concentrations of the drug treatment group, Prism8.0 software was used to fit a drug inhibition rate curve, and a half inhibitory concentration IC₅₀ of salvianolic acid C (Sal-C) to inhibit entering of the SARS-CoV-2 pseudovirus into a target cell was calculated to be 5.90 µM.

### Embodiment 10 Cytotoxicity detection of salvianolic acid C

1. Method:
   1) Cell inoculation:
      Cells Vero-E6 and 293T/ACE2 in a logarithmic growth period were taken, cell densities thereof were adjusted to 1^{∗}10^4 cells/well, and 100 µL/well thereof were respectively inoculated on a 96-well plate and cultured overnight.
   2) Drug concentration design:
      Before administration, a drug was diluted to nine concentration gradients with a DMEM medium containing fetal bovine serum of 2% of a total volume twice multiple proportions, and an initial concentration was set to 200 µM (200, 100, 50, 25, 12.5, 6.25, 3.125, 1.5625, 0.78125 µM), and 100 µL per well of a diluted drug was taken to be added into the cells Vero-E6 and 293T/ACE2 in the 96-well plate in 1), with a final volume of 200 µL per well. Triplicate wells were set for each drug concentration. A DMSO solvent treatment group was used as a blank control.
   3) Absorbance detection:
      After incubation in an incubator for 48 hours, 10 µL of a CCK-8 working solution was added into each well, and incubation continued to be conducted in the incubator for 3 hours. The absorbance at 450 nm was measured by a microplate reader.
   4) According to measured OD values, survival rates of the cells Vero-E6 and 293T/ACE2 under the action of various concentrations of the drug were calculated respectively compared with the control group.
2. Results: as shown in FIG. 12 and FIG. 13;
   Salvianolic acid C (Sal-C) had no obvious toxic effect on the cell Vero-E6 (FIG. 12) and the cell 293T/ACE2 (FIG. 13) at 200 µM and within an effective concentration range.

### Embodiment 11 Detection of inhibitory activity of dihydrotanshinone I on SARS-CoV-2 in vitro

1. Inhibitory activity experiment of drug:
1) A cell Vero-E6 in a logarithmic growth period was taken, with 3^{∗}10^5 cells/well, inoculated into a 48-well plate, and cultured overnight at 37°C with 5% CO₂.
2) Drug pre-incubation: a DMEM medium containing fetal bovine serum of 2% of a total volume was used to dilute a drug. An initial concentration of the drug was set to 10 µM (a solvent was DMSO), the drug was diluted three times multiple proportions, and triplicate wells were set for each concentration, with a total of six drug gradients (10, 3.33, 1.11, 0.37, 0.12, 0.04 µM); a solvent dimethyl sulfoxide (DMSO) was set as a control group, and the control group used the DMEM medium containing the fetal bovine serum of 2% of the total volume for dilution, and the drug was administrated with the same volume of DMSO. After a cell supernatant was removed, 100 µL of a diluted drug was added into each well in an experimental group in the 48-well plate in 1), 100 µL of a diluted DMSO was added in the control group, and they were incubated at 37°C for 1 hour.
3) Virus infection: 5 µL of a SARS-CoV-2 virus diluent was added into each well of the 48-well plate (multiplicity of infection MOI=0.05), and continued to be incubated at 37°C for 1 hour. An infection supernatant was fully removed, and the cell was washed with 200 µL of PBS once. 200 µL of a corresponding concentration of medium containing the drug was re-added into the wells and continued to be cultured for 24 hours, and 150 µL of a cell culture supernatant was taken to be detected. The virus copy number was determined by qRT-PCR.
4) For a specific operation method of virus RNA extraction, referring to Takara MiniBEST Viral RNA/DNA Extraction Kit (Code No. 9766):
a. Virus lysis: 50 µL of a PBS solution (pH7.4) was added into 150 µL of the cell culture supernatant to supplement to 200 µL. 200 µL of a Buffer VGB, 20 µL of Proteinase K and 1.0 µL of Carrier RNA were added, fully mixed, and subjected to a water bath at 56°C for 10 minutes to be fully lysed. 200 µL of absolute ethyl alcohol was added into a lysis solution, and was fully sucked, beaten and mixed evenly.
b. Column chromatography: Spin Column was placed on Collection Tube, a solution was transferred into Spin Column, centrifuged at 12,000 rpm for 2 minutes, and a filtrate was discarded.
c. Washing 1: 500 µL of a Buffer RWA was added into the Spin Column, centrifuged at 12,000 rpm for 1 minute, and a filtrate was discarded.
d. Washing 2: 700 µL of a Buffer RWB was added into the Spin Column, centrifuged at 12,000 rpm for 1 minute, and a filtrate was discarded. (A specified volume of 100% ethyl alcohol has been added into the Buffer RWB). The Buffer RWB was added along a periphery of a tube wall of the Spin Column.
e. Step d was repeated.
f. Spin Column was placed on Collection Tube, and centrifuged at 12,000 rpm for 2 minutes.
g. Elution: Spin Column was placed on a new 1.5 mL RNase free collection tube, and 30 µL of RNase free dH₂O was added to a center of a Spin Column membrane, and stood at a room temperature for 5 minutes. Centrifugation was conducted at 12,000 rpm for 2 minutes for eluting RNA.

5) For a specific operation method of virus RNA reverse transcription, referring to Takara PrimeScriptTM RT reagent Kit with gDNA Eraser (Code No.RR047A):
a. Removing genomic DNA from an eluate: a reaction system was prepared on ice according to the following components.

| **Reagents** | **Volume (µL)** |
|---|---|
| 5^{∗}gDNA Eraser Buffer | 2.0 |
| gDNA Eraser | 1.0 |
| Total RNA | 3.0 |
| RNase Free dHzO | 4.0 |
| Total volume | 10.0 |

A sample was placed at 42°C and reacted for 2 minutes.
b. Reverse transcription reaction:

| **Reagents** | **Volume (µL)** |
|---|---|
| A reaction solution in step 1 | 10.0 |
| PrimeScript RT Enzyme Mix I | 1.0 |
| RT Primer Mix | 1.0 |
| 5×PrimeScript Buffer 2 (for Real Time) | 4.0 |
| RNase Free dH₂O | Supplement to 20.0 |

The sample was incubated at 37°C for 15 minutes and then heated at 85°C for 5 seconds.
6) Detection of the virus copy number by qPCR: referring to Takara TB Green^{®} Premix Ex Taq^{™} II (TliRNaseH Plus, Code, No. RR820A) (using a standard curve method: an RBD plasmid with known copy number was used as a standard, and a specific primer was targeted to RBD). A reaction system was prepared on ice according to the following components.

| **Reagents** | **Volume (µL)** |
|---|---|
| TB Green Premix Ex Taq II (Tli RNaseH Plus) (2X) | 10 |
| Forward Primer (10 µM) | 1 |
| Reverse Primer (10 µM) | 1 |
| ROX Reference Dye (50X) | 0.4 |
| cDNA template | 1 |
| Sterile water | 6.6 |
| Total volume | 20 |

A primer sequence was as follows:
RBD Forward Primer: CAATGGTTTAACAGGCACAGG
RBD Reverse Primer: CTCAAGTGTCTGTGGATCACG
Computer detection: ABI7500 quantitative PCR instrument
Initial denaturation: 95°C, 30 seconds, 1 cycle; PCR amplification: 95°C, 5 seconds, 40 cycles; annealing: 60°C, 30 to 34 seconds; recording.

2. Results: as shown in FIG. 14;

The copy number of each sample was calculated according to a standard curve. An inhibition rate in the drug treatment group was calculated with the copy number in the DMSO group as reference. Then, according to the inhibition rates of different concentrations of the drug treatment group, Prism8.0 software was used to fit a drug inhibition rate curve, and a half effective concentration EC₅₀ of dihydrotanshinone I (DHT) on activity of SARS-CoV-2 was calculated to be 0.64 µM.

### Embodiment 12 Detection of inhibitory activity of Tanshinone I on SARS-CoV-2 in vitro

1. Inhibitory activity experimental method of drug:
   1) A cell Vero-E6 in a logarithmic growth period was taken, with 3^{∗}10^5 cells/well, inoculated into a 48-well plate, and cultured overnight at 37°C with 5% CO₂.
   2) Drug pre-incubation: a DMEM medium containing fetal bovine serum of 2% of a total volume was used to dilute a drug. An initial concentration of the drug was set to 100 µM (a solvent was DMSO), the drug was diluted three times multiple proportions, and triplicate wells were set for each concentration, with a total of six drug gradients (100, 33.33, 11.11, 3.70, 1.23, 0.41 µM); a solvent dimethyl sulfoxide (DMSO) was set as a control group, and the control group used the DMEM medium containing the fetal bovine serum of 2% of the total volume for dilution, and the drug was administrated with the same volume of DMSO. After a cell supernatant was removed, 100 µL of a diluted drug was added into each well in an experimental group in the 48-well plate in 1), 100 µL of a diluted DMSO was added in the control group, and they were incubated at 37°C for 1 hour.
   3), 4). 5), 6) and 7) are the same as **Embodiment 11.**
2. Results: as shown in FIG. 15;

The copy number of each sample was calculated according to a standard curve. An inhibition rate in the drug treatment group was calculated with the copy number in the DMSO group as reference. Then, according to the inhibition rates of different concentrations of the drug treatment group, Prism8.0 software was used to fit a drug inhibition rate curve, and a half effective concentration EC₅₀ of Tanshinone I (T-A) on activity of SARS-CoV-2 was calculated to be 2.98 µM.

### Embodiment 13 Detection of inhibitory activity of cryptotanshinone on SARS-CoV-2 in vitro

1. Inhibitory activity experimental method of drug:
   1) A cell Vero-E6 in a logarithmic growth period was taken, with 3^{∗}10^5 cells/well, inoculated into a 48-well plate, and cultured overnight at 37°C with 5% CO₂.
   2) Drug pre-incubation: a DMEM medium containing fetal bovine serum of 2% of a total volume was used to dilute a drug. An initial concentration of the drug was set to 100 µM (a solvent was DMSO), the drug was diluted three times multiple proportions, and triplicate wells were set for each concentration, with a total of six drug gradients (100, 33.33, 11.11, 3.70, 1.23, 0.41 µM); a solvent dimethyl sulfoxide (DMSO) was set as a control group, and the control group used the DMEM medium containing the fetal bovine serum of 2% of the total volume for dilution, and the drug was administrated with the same volume of DMSO. After a cell supernatant was removed, 100 µL of a diluted drug was added into each well in an experimental group in the 48-well plate in 1), 100 µL of a diluted DMSO was added in the control group, and they were incubated at 37°C for 1 hour.
   3), 4). 5), 6) and 7) are the same as **Embodiment 11.**
2. Results: as shown in FIG. 16;

The copy number of each sample was calculated according to a standard curve. An inhibition rate in the drug treatment group was calculated with the copy number in the DMSO group as reference. Then, according to the inhibition rates of different concentrations of the drug treatment group, Prism8.0 software was used to fit a drug inhibition rate curve, and a half effective concentration EC₅₀ of cryptotanshinone (Cry-T) on activity of SARS-CoV-2 was calculated to be 1.46 µM.

### Embodiment 14 Detection of inhibitory activity of dihydrotanshinone I on entering of SARS-CoV-2 pseudovirus

1. Method:
1) SARS-CoV-2 pseudovirus packaging:
A cell 293T in a logarithmic growth period was taken, with 4^{∗}10^5 cells/mL, 2 mL each well thereof was evenly inoculated in a 6-well plate. Culture was conducted in a 37°C cell incubator with 5% CO₂ for 24 hours. A medium was changed with a fresh medium 0.5 hour before transfection. 100 µL of a blank DMEM medium is used to prepare a plasmid diluent and a transfection reagent (PolyJet) diluent respectively, and a preparation proportion of each well was as follows (plasmid DNA needed to be extracted with an endotoxin-free extraction kit):

| | |
|---|---|
| pNL4-3.Luc.R-E- | 1000 ng |
| pcDNA3.1-SARS-CoV-2-S | 500 ng |
| PolyJet | 6 µL |

A specific preparation method was as follows: a pNL4-3.Luc.R-E-plasmid and a pcDNA3.1-SARS-CoV-2-Sipke plasmid were added into 100 µL of a blank DMEM medium at the same time to be mixed evenly, and PolyJet was diluted and mixed evenly with 100 µL of a blank DMEM medium. The PolyJet diluent was added into the plasmid diluent and mixed evenly, incubated at a room temperature for 10 minutes, and added evenly into the cell HEK-293T. After culture at 37°C for 48 hours, a supernatant virus solution was collected, which was centrifuged at 4,000 rpm for 10 minutes and filtered with a 0.45 µM sterile filter head, and a SARS-CoV-2 pseudovirus was obtained.
2) Pseudovirus inhibition experiment:
A cell 293T (293T/ACE2) overexpressing a SARS-CoV-2 receptor ACE2 in a logarithmic growth period was taken and evenly spread in a 96-well cell plate according to 1^{∗}10^4 cells/well. Culture was conducted in a 37°C cell incubator for 24 hours.

An initial concentration of dihydrotanshinone I was set to 2.5 µM, and was diluted to eight concentration gradients, respectively 2.50, 1.25, 0.625, 0.313, 0.156, 0.078, 0.039, 0.020 µM, with a DMEM medium containing fetal bovine serum of 2% of a total volume twice multiple proportions. A DMSO solvent control group was set with a volume of 60 µL per well and triplicate wells for each concentration. 60 µL of a pseudovirus solution was added into a diluted drug and acted at a room temperature for 30 minutes, and 100 µL/well thereof was taken to be added into the cell 293T/ACE2 and cultured at 37°C for 48 hours. The medium was removed. The cell was washed with 100 µL/well of sterile PBS (pH7.4) once. 50 µL of a 1X cell lysis solution was added into each well, and lysed under oscillation at a room temperature for 15 minutes. 40 µL/well of a lysis supernatant was transferred into a 96-well white ELISA plate. An equal volume of a diluted luciferase substrate was added according to the instructions of a single luciferase detection kit, a fluorescence value was immediately detected by a microplate reader, and according to the fluorescence value, the activity of dihydrotanshinone I to inhibit absorption and entering of the virus was judged. An inhibition rate was calculated according to a corresponding relationship between the fluorescence value and a drug concentration. A curve was drawn, and a half inhibitory concentration IC₅₀ of dihydrotanshinone I was calculated.

2. Results: as shown in FIG. 17;
An inhibition rate in the drug treatment group was calculated according to the fluorescence value with the DMSO group as a control. Then, according to the inhibition rates of different concentrations of the drug treatment group, Prism8.0 software was used to fit a drug inhibition rate curve, and a half inhibitory concentration IC₅₀ of dihydrotanshinone I (DHT) to inhibit entering of the SARS-CoV-2 pseudovirus into a target cell was calculated to be 0.68 µM.

### Embodiment 15 Cytotoxicity detection of dihydrotanshinone I, Tanshinone I and cryptotanshinone

1. Method:
   1) Cell inoculation:
      Cells Vero-E6 and 293T/ACE2 in a logarithmic growth period were taken, cell densities thereof were adjusted to 1^{∗}10^4 cells/well, and 100 µL/well thereof were respectively inoculated on a 96-well plate and cultured overnight.
   2) Drug concentration design:
      Cell Vero-E6: before administration, a drug was diluted to eight concentration gradients with a DMEM medium containing fetal bovine serum of 2% of a total volume three times multiple proportions, and an initial concentration of the dihydrotanshinone I was set to 50 µM (50, 16.67, 5.56, 1.85, 0.62, 0.21, 0.07, 0.02 µM); an initial concentration of the Tanshinone I was set to 100 µM (100, 50, 16.67, 5.56, 1.85, 0.62, 0.21, 0.07 µM); an initial concentration of the cryptotanshinone was set to 100 µM (100, 50, 16.67, 5.56, 1.85, 0.62, 0.21, 0.07 µM); and 100 µL per well of a diluted drug was taken to be added into the cell Vero-E6 in the 96-well plate in 1), with a final volume of 200 µL per well. Triplicate wells were set for each drug concentration. A DMSO solvent treatment group was used as a blank control.
      Cell 293T/ACE2: before administration, the drug was diluted to eight concentration gradients with a DMEM medium containing fetal bovine serum of 2% of a total volume twice multiple proportions, and an initial concentration of the dihydrotanshinone I was set to 10 µM (10, 5, 2.5, 1.25, 0.63, 0.31, 0.16, 0.08 µM). 100 µL per well of a diluted drug was taken to be added into the cell 293T/ACE2 in the 96-well plate in 1), with a final volume of 200 µL per well. Triplicate wells were set for each drug concentration. A DMSO solvent treatment group was used as a blank control.
   3) Absorbance detection:
      After incubation in an incubator for 48 hours, 10 µL of a CCK-8 working solution was added into each well, and incubation continued to be conducted in the incubator for 3 hours. The absorbance at 450 nm was measured by a microplate reader.

### 2. Results:

As shown in FIG. 18, dihydrotanshinone I (DHT) had no obvious toxic effect on the cell Vero-E6 at 10 µM and within an effective concentration range.

As shown in FIG. 19, dihydrotanshinone I (DHT) had no obvious toxic effect on the cell 293T/ACE2 at 2.5 µM and within an effective concentration range.

As shown in FIG. 20, Tanshinone I (T-A) had no obvious toxic effect on the cell Vero-E6 at 100 µM and within an effective concentration range.

As shown in FIG. 21, cryptotanshinone (Cry-T) had no obvious toxic effect on the cell Vero-E6 at 100 µM and within an effective concentration range.

The above are only specific implementations of the present invention, not all implementations. Any equivalent transformation of the technical solution of the present invention by ordinary skill in the art through reading the specification of the present invention is covered by the claims of the present invention.

## Claims

1. A use of an active ingredient of *Salvia miltiorrhiza* Bunge or a pharmaceutically acceptable salt thereof in preparation of an antiviral drug, **characterized in that**, the active ingredient of *Salvia miltiorrhiza* Bunge is salvianic acid A, salvianolic acid B, salvianolic acid C, dihydrotanshinone I or a dihydrotanshinone I analogue.

2. A use of an active ingredient of *Salvia miltiorrhiza* Bunge or a pharmaceutically acceptable salt thereof in preparation of a drug that inhibits a virus from entering a target cell, **characterized in that**, the active ingredient of *Salvia miltiorrhiza* Bunge is salvianic acid A, salvianolic acid B, salvianolic acid C, dihydrotanshinone I or a dihydrotanshinone I analogue.

3. The use according to claim 1 or 2, **characterized in that**, the dihydrotanshinone I analogue is tanshinone I or cryptotanshinone.

4. The use according to claim 1 or 2, **characterized in that**, when the active ingredient of *Salvia miltiorrhiza* Bunge is the salvianolic acid B, the virus is HIV-1, SARS-CoV, SARS-CoV-2 or MERS-CoV.

5. The use according to claim 1 or 2, **characterized in that**, when the active ingredient of *Salvia miltiorrhiza* Bunge is the salvianic acid A, the salvianolic acid C, the dihydrotanshinone I or the dihydrotanshinone I analogue, the virus is a coronavirus.

6. The use according to claim 5, **characterized in that**, when the active ingredient of *Salvia miltiorrhiza* Bunge is the dihydrotanshinone I or the dihydrotanshinone I analogue, the virus is HCoV-229E, HCoV-OC43, HCoV-NL63, HCoV-HKU1, SARS-CoV, SARS-CoV-2 or MERS-CoV.

7. The use of according to claim 5, **characterized in that**, the virus is SARS-CoV-2.

8. A use of an active ingredient of *Salvia miltiorrhiza* Bunge or a pharmaceutically acceptable salt thereof in preparation of a drug that inhibits virus-cell fusion mediated by a viral S protein, **characterized in that**, the active ingredient of *Salvia miltiorrhiza* Bunge is salvianolic acid B.

9. A use of an active ingredient of *Salvia miltiorrhiza* Bunge or a pharmaceutically acceptable salt thereof in preparation of a drug that inhibits formation of a six-helix structure by fusion regions HR1 and HR2 of a subunit S2 of a viral S protein, **characterized in that**, the active ingredient of *Salvia miltiorrhiza* Bunge is salvianolic acid B.

10. An anti-viral pharmaceutical composition, **characterized in that**, the anti-viral pharmaceutical composition contains an active ingredient of *Salvia miltiorrhiza* Bunge or a pharmaceutically acceptable salt thereof as an active substance, and the active ingredient of *Salvia miltiorrhiza* Bunge is salvianic acid A, salvianolic acid B, salvianolic acid C, dihydrotanshinone I or a dihydrotanshinone I analogue.

11. A pharmaceutical composition for inhibiting a virus from entering a target cell, **characterized in that**, the pharmaceutical composition contains an active ingredient of *Salvia miltiorrhiza* Bunge or a pharmaceutically acceptable salt thereof as an active substance, and the active ingredient of *Salvia miltiorrhiza* Bunge is salvianic acid A, salvianolic acid B, salvianolic acid C, dihydrotanshinone I or a dihydrotanshinone I analogue.

12. The pharmaceutical composition according to claim 10 or 11, **characterized in that**, when the active ingredient of *Salvia miltiorrhiza* Bunge is the salvianolic acid B, the virus is HIV-1, SARS-CoV, SARS-CoV-2 or MERS-CoV.

13. The pharmaceutical composition according to claim 10 or 11, **characterized in that**, when the active ingredient of *Salvia miltiorrhiza* Bunge is the salvianic acid A, the salvianolic acid C, the dihydrotanshinone I or the dihydrotanshinone I analogue, the virus is a coronavirus.

14. The pharmaceutical composition according to claim 10 or 11, **characterized in that**, when the active ingredient of *Salvia miltiorrhiza* Bunge is the dihydrotanshinone I or the dihydrotanshinone I analogue, the virus is HCoV-229E, HCoV-OC43, HCoV-NL63, HCoV-HKU1, SARS-CoV, SARS-CoV-2 or MERS-CoV.

15. The pharmaceutical composition according to claim 13, **characterized in that**, the virus is SARS-CoV-2.

16. The pharmaceutical composition according to claim 10 or 11, **characterized in that**, the pharmaceutical composition is an injection preparation or an oral preparation.

17. The pharmaceutical composition according to claim 16, **characterized in that**, the pharmaceutical composition is a set prescription preparation injection, a set prescription preparation tablet, a set prescription preparation capsule, a set prescription preparation pill or a set prescription preparation drop pill.

18. A pharmaceutical composition for inhibiting virus-cell fusion mediated by a viral S protein, **characterized in that**, the pharmaceutical composition contains salvianolic acid B or a pharmaceutically acceptable salt thereof as an active substance.

19. A pharmaceutical composition for inhibiting formation of a six-helix structure by fusion regions HR1 and HR2 of a subunit S2 of a viral S protein, **characterized in that**, the pharmaceutical composition contains salvianolic acid B or a pharmaceutically acceptable salt thereof as an active substance.

20. The pharmaceutical composition according to claim 18 or 19, **characterized in that**, the virus is HIV-1, SARS-CoV, SARS-CoV-2 or MERS-CoV.

21. The pharmaceutical composition according to claim 18 or 19, **characterized in that**, the pharmaceutical composition is an injection preparation or an oral preparation.

22. The pharmaceutical composition according to claim 21, **characterized in that**, the pharmaceutical composition is a set prescription preparation injection, a set prescription preparation tablet, a set prescription preparation capsule, a set prescription preparation pill or a set prescription preparation drop pill.
